# EUROPEAN PATENT APPLICATION

(11) **EP 4 734 117 A1**
(43) Date of publication of application: **29.04.2026**
(21) Application number: 25211013.5
(22) Date of filing: 24.10.2025
(51) Int. Cl.: G16B 30/00, G16B 50/50

(54) **METHOD AND SYSTEM FOR REAL-TIME PROCESSING OF RAW DATA FROM NANOPORE GENE SEQUENCING**

(30) Priority: 25.10.2024 CN 202411498982
(71) Applicant: Geneus Technologies (Chengdu) Co., Ltd., Chengdu, Sichuan 610041 (CN)
(72) Inventor: LONG, Wendi, Chengdu, 610041 (CN); JIANG, Wanhui, Chengdu, 610041 (CN); JIANG, Ke, Chengdu, 610041 (CN)
(74) Representative: Wenzel Nemetzade Warthmüller Patentanwälte Part mbB

(57) **Abstract**

A method and system for real-time processing of raw data from nanopore gene sequencing is provided. In the method, preprocessing and rectangle rotation on raw data obtained from gene sequencing can be performed to convert the data into a continuous single nanopore data structure with a specifiable length. Then, compression on the data after the preprocessing and the rectangle rotation is performed to eliminate invalid data. The data processing method designed in this way as well as a digital circuit and a hardware architecture which are realized in cooperation with the data processing method are particularly suitable for compressing the raw data in the field of large-throughput nanopore gene sequencing. Using the solution of the present disclosure can solve harsh requirements of the prior art for a transmission bandwidth, a computing performance, and a storage medium bandwidth and capacity, and greatly reduce a hardware cost. Since a performance requirement for a back end is reduced, miniaturization of a device can be realized, overall power consumption of the device is reduced, and also a further improvement of the sequencing throughput is facilitated. Meanwhile, since a data amount is reduced, a back end resolving server can perform transmission and resolving at the same time, and a sequencing time can be shortened relatively.

## Description

### Field of the Invention

The present disclosure relates to a biological nucleic acid sequencing technology, and more particularly to a method and system for real-time processing of raw data from nanopore gene sequencing.

### Background of the Invention

In the current field of nucleotide sequencing technologies, there are mainly second generation sequencing technologies represented by synthesis sequencing, such as bridge sequencing and pyrosequencing, third generation sequencing technologies represented by single molecule fluorescent sequencing, and fourth generation sequencing technologies with nanopores as technical features. Nanopore-based nucleic acid sequencing realizes spanning from traditional optical signal sequencing to digital electrical signal sequencing, and has a major principle that some transmembrane proteins, such as bacterial toxin (α-hemolysin), can form stable channels (called nanopores) with diameters of about 1-2 nanometers on a phospholipid membrane, and single-stranded DNA or RNA molecules can spontaneously pass through the nanopores in an electric field due to charged properties and cause a change of resistance of the nanopores during passing to generate a so-called blocking current. Due to differences of chemical structures of four different bases A, T or U, C and G of DNA or RNA, blocking influences on the current generated when the bases pass through the nanopores have recognizable differences, and respective corresponding feature blocking currents are generated. Accurate detection of the feature blocking current can determine a type of the corresponding base, thereby determining a nucleic acid sequence.

Large-throughput nanopore sequencing is defined as integrating a plurality of nanopores (ten thousands level or more) in one chip package, current data collected by the nanopores has the characteristic that the data of a single nanopore is correlated on a time axis, and the data between the pores has no strong correlation, such characteristic causes that data processing difficulty is exponentially increased along with an increase of a throughput. Currently, the throughput of nanopore gene sequencing is increased to a million level, and under the conditions of a 8-bit sampling resolution and a 1kSa/s (1 thousand times of sampling per second) sampling rate (the sampling rate cannot be reduced by limitations of characteristics of a biochemical system), an original sampled data stream of a GB/s (gigabyte per second) level will be generated (if the throughput is increased to ten millions, data of 10GB/s or more will be generated). Such a large amount of data brings a great challenge to receiving, calculating and storing of a back end platform, and high-end computing cards (e.g., A6000 and A100) and large servers are often required for performing resolving.

Solutions for realizing high throughput sequencing at present basically adopt a transmission interface with a higher transmission bandwidth, a resolving server platform with a higher performance and a storage medium with a higher storage bandwidth and a higher storage capacity to meet design requirements, and meanwhile, calculation, transmission and storage tasks are subjected to time sharing processing, that is, transmission and storage are firstly performed, and then, data processing and calculation are performed. Regarding data compression, data compression in the existing gene sequencing field is performed after gene fragment data is resolved, and the problem that the amount of the original data is too large before resolving cannot be solved.

### Summary

As described above, since the sampling rate of the raw data is much larger than an actual event occurrence rate, a proportion of invalid data is quite large and 90% or more. Moreover, since the amount of the raw data is too large, a storage medium needs to be quite large, such as a high-performance hard disk of tens TBs or more. Meanwhile, since the amount of the raw data is too large and a resolving time needs to be shortened as soon as possible, a transmission rate needs to be quite high. However, in the large amount of raw data, single pore data is not continuous, and the resolving time needs to be shortened as soon as possible, such that resolving hardware resources of a back end need to be quite high, such as an architecture of multiple GPUs plus multiple CPUs. The above problems can cause a whole sequencer to have an apparatus hardware with quite complexity, a large volume, high power consumption and a high price cost; eventually, further expansion of the throughput is inconvenient, mounting, transportation, or the like, are inconvenient, and a sequencing time effect is relatively low.

Therefore, a data processing method as well as a digital circuit and a hardware architecture which are realized in cooperation with the data processing method are designed, and particularly suitable for compressing the raw data in the field of large-throughput nanopore gene sequencing.

According to a first aspect of the present disclosure, there is provided a method for processing raw data from gene sequencing. The method can include: performing preprocessing and rectangle rotation on raw data obtained from gene sequencing to convert the raw data into a continuous single nanopore data structure with a specifiable length; and performing compression on the data after the preprocessing and the rectangle rotation to eliminate invalid data.

In the method according to the first aspect of the present disclosure, the preprocessing and the rectangle rotation may further include: performing bit deletion on the data to satisfy byte alignment; performing ordering and alignment on the data according to nanopore channels, and selecting specific channels for performing marking, so as to facilitate subsequent data checking; and rectangle rotating the data ordered according to the nanopore channels into the continuous single nanopore data structure with the specifiable length.

Preferably, the performing bit deletion on the data to satisfy byte alignment may further include: performing bit reduction on the data in a manner of converting the data from absolute values to relative values.

Preferably, the continuous single nanopore data structure with the specifiable length can present contextual information of the data to facilitate subsequent data compression.

Preferably, the specifiable length may be adjusted according to a size of hardware-integrated DDR particles and a throughput amount of a sequencing chip. In addition, read-write addresses of the data during the rectangle rotation can be performed randomized processing according to different DDR capacities and DDR bank sizes.

In the method according to the first aspect of the present disclosure, the compression may further include: adopting a compression encoding algorithm to eliminate the invalid data.

In the method according to the first aspect of the present disclosure, the invalid data may include invalid data without sequencing blocking and invalid data with sequencing blocking.

Preferably, the compression may further include: eliminating invalid continuous current value data when sequencing blocking exists and sequencing blocking does not exist.

In the method according to the first aspect of the present disclosure, the compression may further include: compressing the data after the preprocessing and the rectangle rotation to a state that the data can directly correspond to fragment sequence data of a base type.

In the method according to the first aspect of the present disclosure, after the data compression is performed, caching may be performed on the compressed data for back end resolving.

According to a second aspect of the present disclosure, there is provided a nucleic acid sequencing method. The method can include: obtaining data of a current signal passing through a nanopore; performing processing the obtained data of the current signal according to the method for processing raw data from gene sequencing in the first aspect of the present disclosure; performing resolving according to the compressed data to determine types of bases passing through the nanopore chronologically; and determining a nucleic acid sequence according to the determined types of the bases passing through the nanopore chronologically.

According to a third aspect of the present disclosure, there is provided an apparatus for processing raw data from gene sequencing. The apparatus can include: a data preprocessing and rectangle rotation unit configured to perform preprocessing and rectangle rotation on raw data obtained from gene sequencing to convert the raw data into a continuous single nanopore data structure with a specifiable length; and a data compressing unit configured to perform compression on the data after the preprocessing and the rectangle rotation to eliminate invalid data.

According to a fourth aspect of the present disclosure, there is provided a nucleic acid sequencing system. The system may include: a current acquiring apparatus configured to obtain data of a current signal passing through a nanopore; the apparatus for processing raw data from gene sequencing according to the third aspect of the present disclosure, configured to perform processing on the obtained data of the current signal; a base type determining apparatus configured to perform resolving according to the compressed data after processing by the apparatus for processing gene sequencing original data, and to determine types of bases passing through the nanopore chronologically; and a nucleic acid sequence determining apparatus configured to determine a nucleic acid sequence according to the types of the bases chronologically passing through the nanopore determined by the base type determining apparatus.

According to a fifth aspect of the present disclosure, there is provided a non-transitory computer-readable storage medium for storing a computer program. The computer program includes instructions. The instructions, when executed by a processor of an electronic device, cause the electronic device to perform the method for processing raw data from gene sequencing in the first aspect of the present disclosure or the nucleic acid sequencing method in the second aspect of the present disclosure.

The solution of the present disclosure can solve harsh requirements of the prior art for a transmission bandwidth, a computing performance, and a storage medium bandwidth and capacity, and greatly reduce a hardware cost. Since a performance requirement for the back end is reduced, miniaturization of a device can be realized, overall power consumption of the device is reduced, and a further improvement of the sequencing throughput is facilitated. Meanwhile, since the data amount is reduced, a back end resolving server can perform transmission and resolving at the same time, and a sequencing time can be shortened relatively.

### Brief Description of the Drawings

The present disclosure will be more fully understood from the following detailed description in combination with the accompanying drawings in which like elements are numbered in a similar manner, and in the drawings:
FIG. 1 illustratively depicts a method for processing raw data from gene sequencing according to an embodiment of the present disclosure.
FIG. 2 shows a flow of processing of raw data from gene sequencing depicted in conjunction with hardware.
FIG. 3 is an exemplary view of a 9-throughput sequencing chip.
FIG. 4 is an exemplary view of the raw data.
FIG. 5 is an exemplary view of channel alignment.
FIG. 6 is an exemplary view of channel marking.
FIG. 7 is an exemplary view of data rectangle rotation.
FIG. 8 is an exemplary view of data compression.
FIG. 9 is a block diagram of a first embodiment of a circuit system for implementing the solution of the present disclosure.
FIG. 10 is a block diagram of a second embodiment of the circuit system for implementing the solution of the present disclosure.
FIG. 11 illustratively depicts a nucleic acid sequencing method according to the present disclosure.
FIG. 12 illustratively depicts a schematic block diagram of an apparatus for processing raw data from gene sequencing according to the present disclosure.
FIG. 13 illustratively depicts a schematic block diagram of a nucleic acid sequencing system according to the present disclosure.

### Detailed Description

The technical solution of the present disclosure will be described in further detail below with reference to the embodiment and drawings, but the present disclosure is not limited to the following embodiment.

### Method for processing raw data from gene sequencing

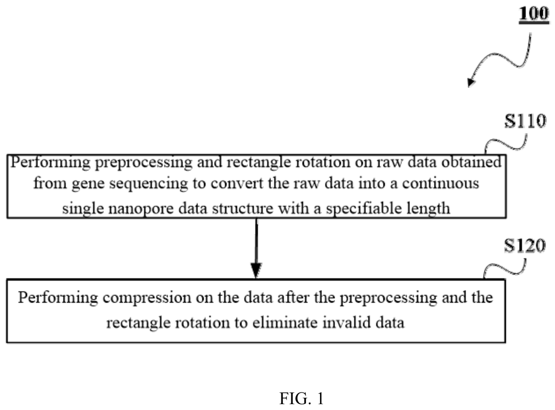

FIG. 1 illustratively depicts a method for processing raw data from gene sequencing according to an embodiment of the present disclosure.

As shown in FIG. 1, the method 100 for processing raw data from gene sequencing according to the embodiment of the present disclosure begins with a step S110, in this step: preprocessing and rectangle rotation is performed on raw data obtained from gene sequencing to convert the raw data into a continuous single nanopore data structure with a specifiable length.

According to a preferred embodiment of the present disclosure, the step S110 may further include: performing bit deletion on the obtained raw data from gene sequencing to satisfy byte alignment; performing channel marking and ordering on the raw data according to nanopores; and rectangle rotating the data ordered according to nanopore channels into the continuous single nanopore data structure with the specifiable length.

Next, in a step S120: compression is performed on the data after the preprocessing and the rectangle rotation to eliminate invalid data.

According to a preferred embodiment of the present disclosure, in the step S120, the invalid data may be eliminated by using a compression encoding algorithm. The data compression algorithms include, but are not limited to, the following algorithms: differential encoding, Huffman encoding, LZW encoding, or the like. A more detailed explanation is given below.

Furthermore, the invalid data described herein may include invalid data without sequencing blocking and invalid data with sequencing blocking. A more detailed explanation is given below.

In the step S120, an ideal state of data compression is as follows: the data after the preprocessing and the rectangle rotation is compressed to a state that the data can directly correspond to fragment sequence data of a base type.

According to a preferred embodiment of the present disclosure, after the step S120, that is, after performing compression on the data, caching may be performed on the compressed data, in order for back end resolving.

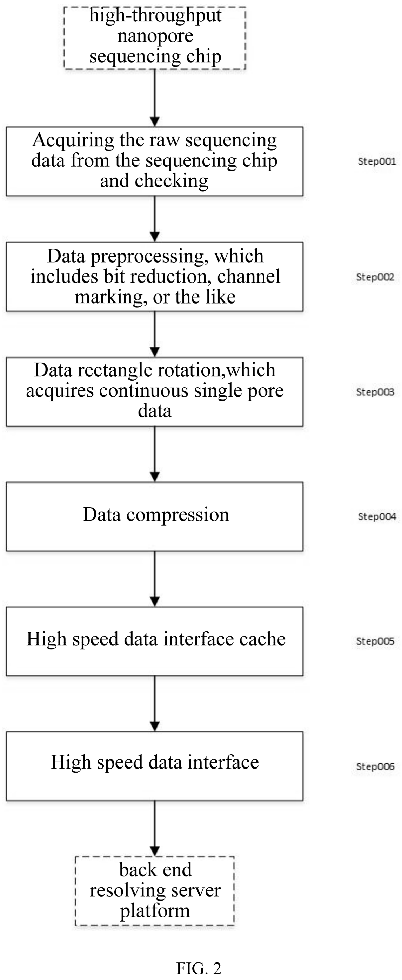

FIG. 2 shows a flow of processing of the raw data from gene sequencing depicted in a manner of in conjunction with hardware. The solid line part of FIG. 2 is an implementation flow of the method according to the present disclosure, the dotted line part is upstream and downstream devices for implementing the present disclosure, a front end is a high-throughput nanopore sequencing chip (sensor), a back end is a resolving server platform, and when a data compression rate reaches a certain degree, devices, such as GPUs, can be considered to be used as a resolving server, which further reduces a volume of the device.

As shown in FIG. 2, in combination with the hardware, a subject of the present disclosure includes 6 steps: i.e., Step001 to Step006.
1. Step001, implementing acquisition of the raw sequencing data from the sequencing chip and checking, which includes implementation of a sequencing chip interface protocol and data correctness checking.
2. Step002, performing data preprocessing, which includes bit reduction, channel alignment and marking, or the like.

The bit reduction means that, bit deletion of special algorithm processing is mainly performed on the sequencing data to realize first-step data compression, and to enable the data to satisfy byte alignment, thereby facilitating computer processing.

The channel alignment and marking mean that, the data is ordered and aligned according to the nanopores, and specific channels are selected to be performed marking, so as to facilitate subsequent data checking.

An example explanation regarding data preprocessing is given below.

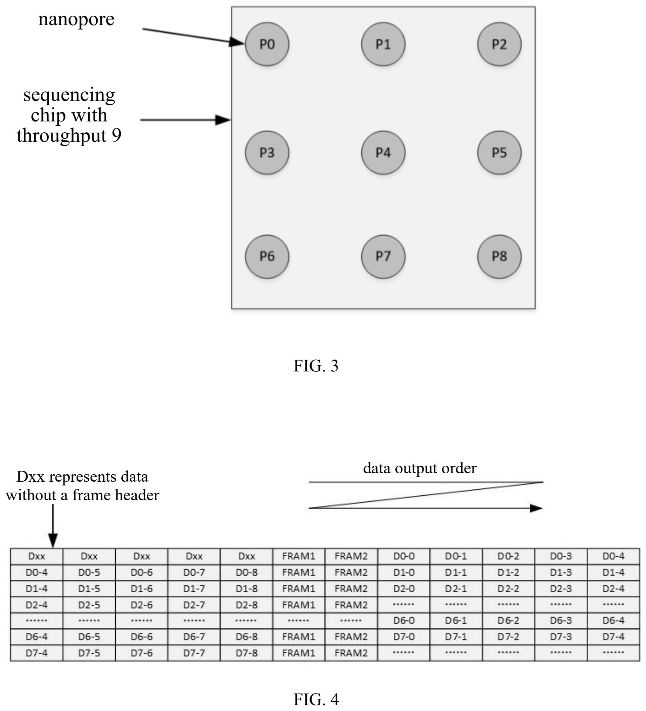

For easy understanding, assuming that a throughput of the sequencing chip is 9 (actual throughput is in a million level), the nanopores of the sequencing chip are arranged as shown in FIG. 3, and after sequencing is started, the sampled data is sequentially output according to P0 to P8 after a frame header is inserted, and the data of 9 channels serves as one frame.

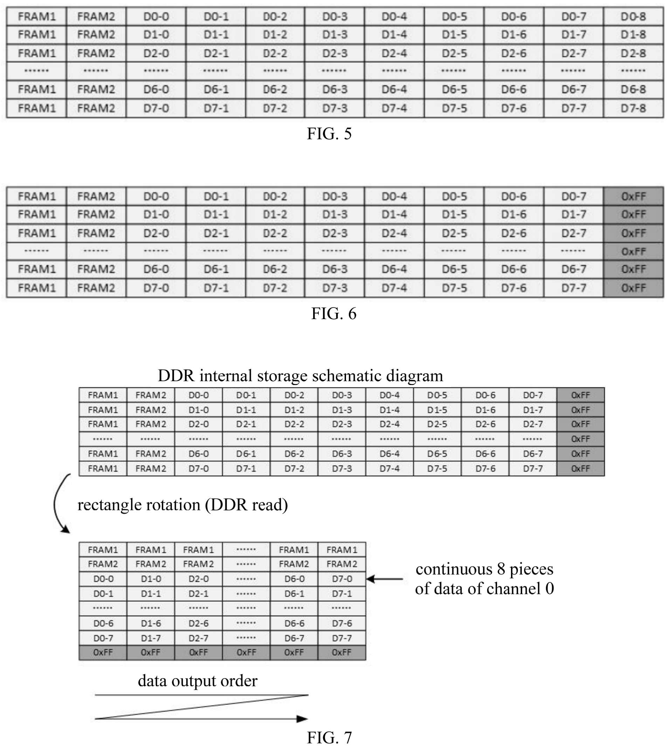

Due to a timing problem, start data received by the system may lack the frame header, such as Dxx data shown in FIG. 4. The data in FIG. 4 is presented in a Dm-n format and represents the sampled data of the n-th nanopore of the m-th frame; FRAM1 and FRAM2 represent data frame headers. Therefore, the system needs to discard the data without the frame header, and find first frame header to alignment data, and the aligned data is shown in FIG. 5.

For convenience of system debugging, the data of the last channel is particularly marked by hexadecimal data 0xFF, i.e., the channel marking function, as shown in FIG. 6.

The bit reduction function means that data bits larger than 8 bits are deleted in a special way, and since bit width of an ADC (digital-to-analog converter) of the sequencing chip are mostly 9 or 10-bit unsigned data, taking 9 bits as an example, a data expression range is from 0 to 511, wherein 256 is a direct current value, and useful information of the data is a difference value between an actual data value and the direct current value, an absolute value is taken after the direct current value 256 is subtracted from the actual value, and an obtained value can be expressed by using a single byte, so as to achieve the bit reduction function. For example, data obtained after the bit reduction processing of the original data 140, 245, 256, 260 and 300 is 116, 11, 0, 4 and 44. That is, performing bit reduction (i.e., compression) on the data in a manner of converting the data from absolute values into relative values.

3. Step003: data rectangle rotation, acquiring continuous single pore data which facilitates implementation of a subsequent compression algorithm.

The data rectangle rotation means that the data ordered according to the nanopore channels is converted into a continuous single pore data structure with a specifiable length that is easy to process. The processing is one of the core steps of the solution of the present disclosure, is realized depending on a specific hardware structure and a digital circuit algorithm, and has the characteristics of a high memory access bandwidth, a real-time performance, or the like.

An example explanation regarding data rectangle rotation is given below.

The data rectangle rotation is still illustrated with the throughput 9 and the data length 8 as an example. The rectangle rotation manner is shown in FIG. 7. The data in FIG. 7 is still presented in a Dm-n format and represents the sampled data of the n-th nanopore of the m-th frame; FRAM1 and FRAM2 represent data frame headers. The rectangle rotation mainly uses DDR as a rectangle rotation cache, and the rectangle rotation is completed through one specific access process. As shown in the upper part of FIG. 7, data is written into the DDR according to write address sequences. For example, the write address sequences of various rows in the upper part of FIG. 7 are:
write address: 0, 1, ..., 10
write address: 11, 12, ..., 21
write address: 22, 23, ..., 32
write address: ...
write address: 66, ..., 76
write address: 77, ..., 87

After 8 frames of data are cached, as shown in the lower part of FIG. 7, the cached data in the DDR is read out according to read address jump sequences, and each row of the read data is continuous data with a single pore length of 8. For example, the read address jump sequences of all rows in the lower part of FIG. 7 are:
read address: 0, 11, 22, ..., 66, 77
read address: 1, 12, 23, ..., 67, 78
read address: 2, 13, 24, ..., 68, 79
read address: 3, 14, 25, ..., 69, 80
read address: ...
read address: 8, 19, 30, ..., 74, 85
read address: 9, 20, 31, ..., 75, 86
read address: 10, 21, 32, ..., 76, 87

Such a data structure can present certain contextual information and can satisfy the implementation of the subsequent compression algorithm. The actual length of the rectangle rotation may be performed adjustment according to a size of hardware-integrated DDR particles and the throughput amount of the sequencing chip, the throughput amount is on thousands level in generally. Due to characteristics of the DDR itself, the address jump sequence needs a long addressing time when crossing DDR banks, thus such an operating mode has a quite low bandwidth at a read end, the read and write addresses need to be further randomized processing, bank-crossing probabilities of the read end and a write end are ensured to be equivalent, read and write bandwidths are balanced (a redundant write bandwidth is sacrificed, and the read bandwidth is improved), and the algorithm needs to be adjusted according to different DDR capacities and bank sizes during the address randomized processing.

4. Step004: data compression, which has a main function that the invalid data in the raw sequencing data is eliminated through the compression encoding algorithm to obtain a small amount of valid data which can be accurately resolved by the back end resolving server platform, and an optimal compression state can achieve directly outputting fragment sequence data of four bases A, G, C and T. The sequencing data mainly includes two kinds of invalid data, i.e., the invalid data without sequencing blocking (defined when no base exists in the nanopore) and the invalid data with sequencing blocking (defined when a base exists in the nanopore, for example, the same base corresponds to a long-time continuous blocking current, but resolving does not need such a long time for identification), and the invalid data is mainly shown as continuous similar current values (for example, the invalid data with sequencing blocking is a continuous blocking current, and the invalid data without sequencing blocking is a baseline current), and can be deleted on a premise that without influencing sequencing accuracy. The data compression algorithms include, but are not limited to, the following algorithms: differential encoding, Huffman encoding, LZW encoding, or the like. Data compression does not include storage compression and only performs compressing on data stream.

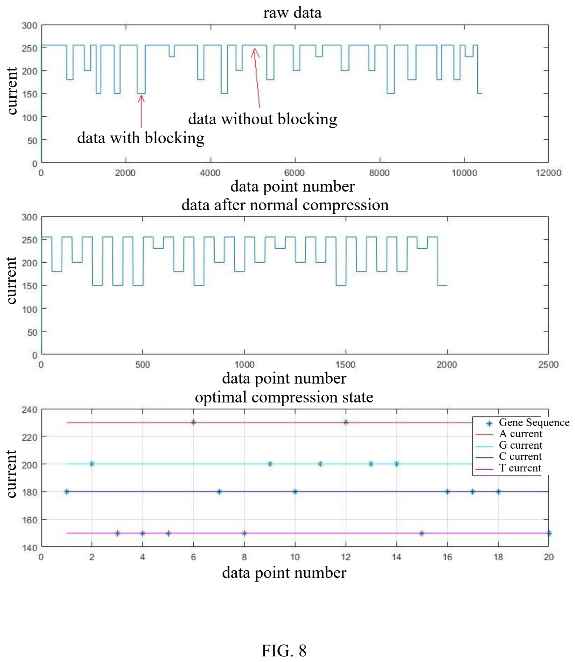

The data compression process may be as shown in FIG. 8.

A relatively ideal data model is shown in FIG. 8, and there is without sampling noise and without direct current drift in the model. The invalid data is shown as invalid continuous current values with and without blocking, and each time of blocking is a sequencing event indicating that one base is determined. As shown in the lower graph of FIG. 8, 20 bases generate more than 10,000 pieces of raw sequencing data, which can be compressed to over 2,000 pieces, and only 20 pieces of data after the optimal compression state.

5. Step005: high speed data interface cache, which is mainly configured to cache interface data and assist in finishing an interface protocol, so as to ensure that data loss of the interface caused by congestion is avoided.

6. Step006: high speed data interface, which includes, but is not limited to, Ethernet interfaces of various rates and electrical standards, various versions of PCIe interfaces, and high speed data interfaces of custom protocols.

Embodiments of a circuit system for implementing the solution of the present disclosure are given below.

### First embodiment:

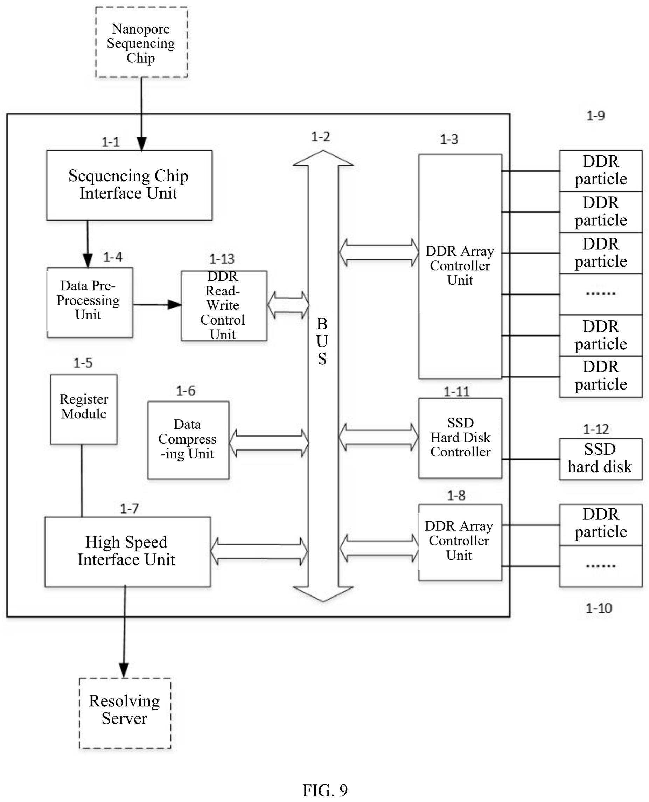

FIG. 9 is a block diagram of a first embodiment of the circuit system for implementing the solution of the present disclosure.

As shown in FIG. 9, a sequencing chip interface unit 1-1 satisfies a data interface protocol of the sequencing chip, receives the original data, and implements the function of Step001.

A storage unit includes DDR array controller units 1-3 and 1-8 and DDR particles 1-9 and 1-10, and is mainly characterized in that hardware includes a plurality of DDR particles, and each DDR controller unit includes one or more independent control channel. The DDR storage particles include DDR2, DDR3, DDR4, DDR5 and other devices.

A data preprocessing unit 1-4 implements the function of Step002.

A DDR read-write control unit 1-13 and the above-described storage units 1-3 and 1-9 achieve the function of Step003, and the DDR read-write control unit 1-13 includes read and write address generation logic required by data rectangle rotation, and meanwhile has address randomization logic to balance the bandwidths of both read and write sides.

A data compressing unit 1-6 implements the function of Step004.

A register module 1-5 is configured to configure working parameters of the overall system.

The DDR array controller unit 1-8 and the DDR particle array 1-10 achieve the function of Step005. A high speed interface unit 1-7 transmits the data to the back end resolving server, protocols thereof including but not limited to an Ethernet interface, a fiber interface, a PCIe interface, or the like, and implements the function of Step006.

An SSD hard disk controller 1-11 controls a large-capacity SSD hard disk 1-12 for local storage of the data.

A bus 1-2 is configured to implement data sharing.

### Second embodiment:

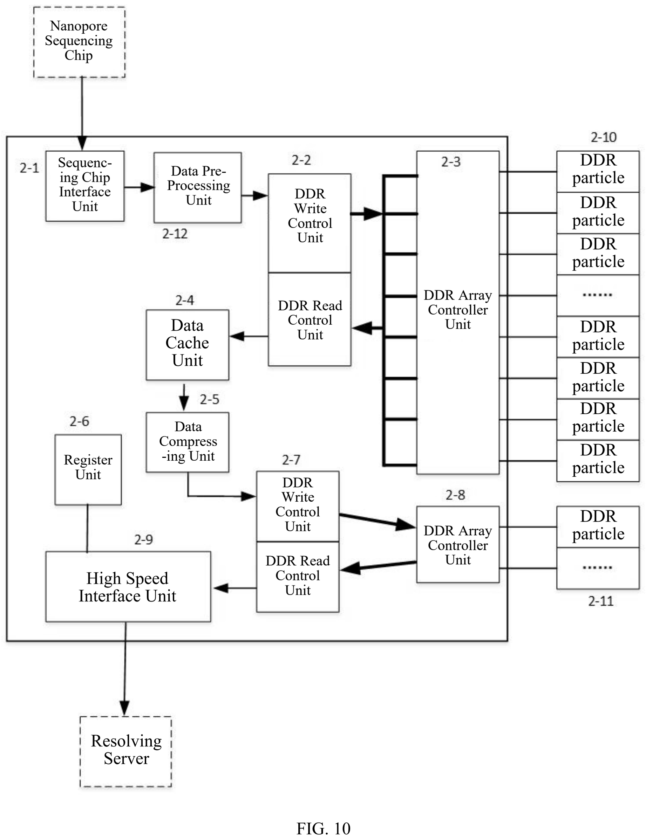

FIG. 10 is a block diagram of a second embodiment of the circuit system for implementing the solution of the present disclosure.

As shown in FIG. 10, a sequencing chip interface unit 2-1 satisfies a data interface protocol of the sequencing chip, receives the original data, and implements the function of Step001.

A storage unit includes DDR array controller units 2-3 and 2-8 and DDR particles 2-10 and 2-11, and is mainly characterized in that hardware includes a plurality of DDR particles, and each DDR controller unit includes one or more independent control channel. The DDR storage particles include DDR2, DDR3, DDR4, DDR5 and other devices.

A register module 2-6 is configured to configure working parameters of the overall system.

A high speed interface unit 2-9 transmits the data to the back end resolving server, protocols thereof including but not limited to an Ethernet interface, a fiber interface, a PCIe interface, or the like, and completes the function of Step006.

The second embodiment is mainly different from the first embodiment in that the sharing bus is not used, a dedicated data channel is used for data interaction, and a larger data bandwidth and data processing capacity can be obtained. Secondly, the large-capacity hard disk is not used for storage due to an improved data processing capacity. Specifically, the data preprocessing function of Step002 is achieved by a data preprocessing unit 2-12. The rectangle rotation function of Step003 for the data after preprocessing is achieved through a DDR write control unit, a DDR read control unit 2-2, a DDR array controller unit 2-3 and a DDR particle 2-10, and wherein the DDR write control unit and the DDR read control unit 2-2 include a write control unit and a read control unit respectively, can achieve a write address and read address generation function required by the rectangle rotation, and have an address randomization capacity to satisfy the read and write bandwidth balance.

The data after the rectangle rotation is cached in a data cache unit 2-4, and a data time sequence is adjusted. Then, the data enters a data compressing unit 2-5 to implement the function of Step004. The compressed data directly interacts with the storage unit (through the DDR array controller unit 2-8) via the DDR read/write control unit 2-7 to achieve the cache function of Step005, and is output to the high speed interface unit 2-9.

Other descriptions:
the digital circuit presented in the above embodiment may be implemented by choosing a programmable logic device (such as an FPGA and a CPLD), or may be designed as a dedicated digital integrated circuit (IC).

### Nucleic acid sequencing method

From the above description, those skilled in the art should appreciate that the present disclosure actually further provides a nucleic acid sequencing method.

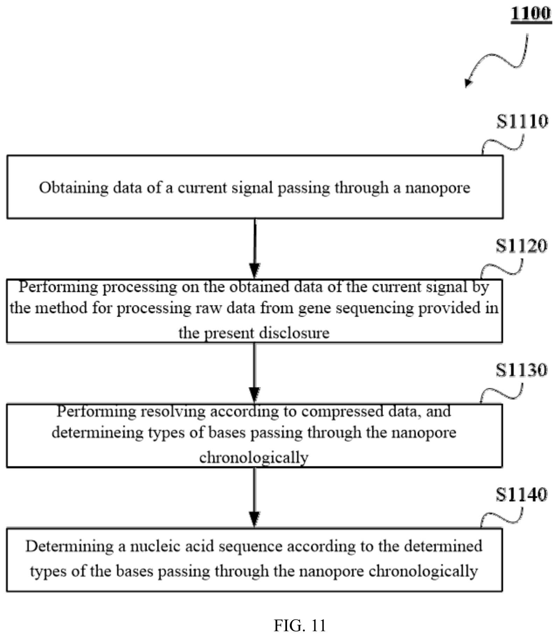

FIG. 11 illustratively depicts the nucleic acid sequencing method according to the present disclosure.

As shown in FIG. 11, the nucleic acid sequencing method 1100 according to the present disclosure begins with step S1110, in this step: data of a current signal passing is obtained through a nanopore.

Next, in step S1120: processing may be performed on the obtained data of the current signal by the method for processing gene sequencing original data provided in the present disclosure.

Then, in step S1130: resolving is performed according to compressed data, and types of bases passing through the nanopore chronologically are determined.

Finally, in step S1140: a nucleic acid sequence is determined according to the determined types of the bases passing through the nanopore chronologically.

### Apparatus for processing gene sequencing original data

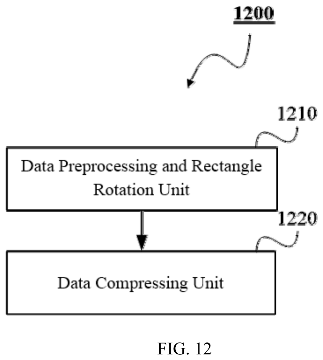

It should be understood by those skilled in the art that an apparatus for processing raw data from gene sequencing can be developed based on the method for processing raw data from gene sequencing according to the present disclosure. FIG. 12 illustratively depicts a schematic block diagram of the apparatus for processing raw data from gene sequencing according to the present disclosure. Specifically, the apparatus 1200 for processing raw data from gene sequencing includes a data preprocessing and rectangle rotation unit 1210 and a data compressing unit 1220.

The data preprocessing and rectangle rotation unit 1210 is configured to perform preprocessing and rectangle rotation on raw data obtained from gene sequencing to convert the raw data into a continuous single nanopore data structure with a specifiable length. The data preprocessing and rectangle rotation unit 1210 corresponds to the step S110 in the method 100.

The data compressing unit 1220 is configured to perform compression on the data after the preprocessing and the rectangle rotation to eliminate invalid data. The data compressing unit 1220 corresponds to the step S120 in the method 100.

It should be understood by those skilled in the art that the above-described apparatus for processing gene sequencing original data 1200 may be implemented by a computer. For example, the following operations may be implemented by executing a computer program: performing preprocessing and rectangle rotation on raw data obtained from gene sequencing to convert the original data into the continuous single nanopore data structure with the specifiable length; and performing compression on the data after the preprocessing and the rectangle rotation to eliminate the invalid data.

That is, although in the apparatus 1200 for processing raw data from gene sequencing according to the present disclosure, various operational functions corresponding to the method for processing raw data from gene sequencing are described as modules or sub-modules, it should be understood by those skilled in the art that such modules or sub-modules may be circuit components or other physical components, or may not be constituted by circuit components or other physical components, but may be functional modules or programmable circuit modules constructed by a computer program.

### Nucleic acid sequencing system

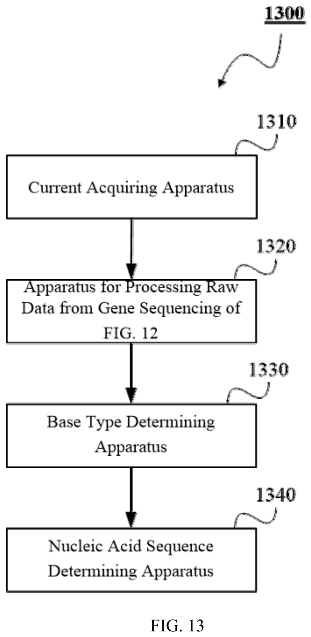

Similarly, those skilled in the art should appreciate that a nucleic acid sequencing system may be developed based on the nucleic acid sequencing method according to the present disclosure. FIG. 13 illustratively depicts a schematic block diagram of the nucleic acid sequencing system according to the present disclosure. Specifically, the nucleic acid sequencing system 1300 includes a current acquiring apparatus 1310, an apparatus 1320 for processing raw data from gene sequencing, a base type determining apparatus 1330, and a nucleic acid sequence determining apparatus 1340.

The current acquiring apparatus 1310 is configured to obtain data of a current signal passing through a nanopore. The current acquiring apparatus 1310 corresponds to the step S1110 in the method 1100.

The apparatus 1320 for processing raw data from gene sequencing is the apparatus for processing raw data from gene sequencing according to the present disclosure, i.e., 1200 in FIG. 12, and it is possible to perform processing on the obtained data of the current signal by the method for processing raw data from gene sequencing provided in the present disclosure. The apparatus 1320 for processing raw data from gene sequencing corresponds to the step S1120 in the method 1100.

The base type determining apparatus 1330 is configured to perform resolving according to compressed data after processing by the apparatus 1320 for processing raw data from gene sequencing, and to determine types of bases passing through the nanopore chronologically. The base type determining apparatus 1330 corresponds to the step S1130 in the method 1100.

The nucleic acid sequence determining apparatus 1340 is configured to determine a nucleic acid sequence according to the types of the bases chronologically passing through the nanopore determined by the base type determining apparatus 1330. The nucleic acid sequence determining apparatus 1340 corresponds to the step S1140 in the method 1100.

It should be understood by those skilled in the art that the above-described nucleic acid sequencing system 1300 may be implemented by a computer. For example, the following operations may be implemented by executing a computer program: obtaining the data of the current signal passing through the nanopore; performing processing on the obtained data of the current signal by the method for processing raw data from gene sequencing provided in the present disclosure; performing resolving according to the compressed data after processing to determine the types of the bases passing through the nanopore chronologically; and determining the nucleic acid sequence according to the types of the bases passing through the nanopore chronologically.

That is, although in the nucleic acid sequencing system 1300 according to the present disclosure, various operational functions corresponding to the nucleic acid sequencing method are described as modules or sub-modules, it should be understood by those skilled in the art that such modules or sub-modules may be circuit components or other physical components, or may not be constituted by circuit components or other physical components, but may be functional modules or programmable circuit modules constructed by a computer program.

### Embodiments based on computer program or instructions

It will be appreciated by those skilled in the art that the method according to the present disclosure may be implemented as a computer program. As mentioned above with reference to the drawings, the method according to the above-mentioned embodiment is performed by one or a plurality of programs, including instructions to cause a computer or processor to perform an algorithm described with reference to the drawings. These programs may be stored and provided to the computer or processor using various types of non-transitory computer readable media. The non-transitory computer readable media include various types of tangible storage media. Examples of the non-transitory computer readable medium include a magnetic recording medium (such as a floppy disk, a magnetic tape, and a hard disk drive), a magneto-optical recording medium (such as a magneto-optical disk), a CD-ROM (a compact disc-read-only memory), a CD-R, a CD-R/W, and a semiconductor memory (such as a ROM, a PROM (a programmable ROM ), an EPROM (an erasable PROM), a flash ROM and a RAM (a random access memory)). Further, these programs may be provided to the computer using various types of transitory computer readable media. Examples of the transitory computer readable medium include an electric signal, an optical signal, and an electromagnetic wave. The transitory computer readable medium may be configured to provide the program to the computer through a wired communication path, such as an electric wire and an optical fiber, or a wireless communication path.

For example, an embodiment of the present disclosure may provide a non-transitory computer-readable storage medium having a computer program stored thereon, the computer program including instructions that, when executed by a processor of an electronic device, cause the electronic device to perform the method for processing raw data from gene sequencing as described before or the nucleic acid sequencing method as described before.

For example, an embodiment of the present disclosure may further provide a computer system including a processor, a memory and a computer program. The computer program is stored in the memory and configured to be executed by the processor. The computer program includes instructions for implementing the method for processing raw data from gene sequencing as described above or the nucleic acid sequencing method as described above.

### Solution summary and beneficial effect

The solution of the present disclosure includes the following core points (which can be considered as technical features that form a distinction from prior art solutions):
1. the present disclosure proposes the preprocessing method of raw sequencing data, the hardware framework for real-time rectangle rotation and the digital circuit algorithm for real-time rectangle rotation;
2. the present disclosure proposes the compression method of the raw sequencing data that has the property that subsequent resolving can be performed without decompression.

The solution of the disclosure has the following beneficial effects and advantages:
1. A dedicated hardware circuit and a specific high-speed data parallel processing algorithm are designed, real-time hardware rectangle rotation of a certain time period, such as 1 second (1,000 points) and 5 seconds (5,000 points), can be realized, a single-pore continuous data slice is output after the rectangle rotation, and the method can greatly save computing power of the server and is a necessary condition for achieving subsequent functions;
2. For the single-pore continuous data slice, a dedicated circuit and algorithm are designed for performing compression of the data, and theoretically, a compression rate of 90% or more (for example, 100MB (megabytes) of data are compressed to be within 10MB) can be realized, and real-time compression is performed, thus effectively reducing the data amount and the transmission bandwidth requirements;
3. The compressed data retains complete sequencing blocking information, resolving can be performed without decompression, gene-sequence-level data can be reached theoretically, and resolving complexity is greatly reduced.

In conclusion, the solution of the present disclosure can solve harsh requirements of the prior art for a transmission bandwidth, a computing performance, and a storage medium bandwidth and capacity, and greatly reduce a hardware cost. Since a performance requirement for the back end is reduced, miniaturization of the device can be realized, overall power consumption of the device can be reduced, and a further improvement of the sequencing throughput is facilitated. Meanwhile, since the data amount is reduced, the back end resolving server can perform transmission and resolving at the same time, and a sequencing time can be shortened relatively.

The embodiments of the present disclosure are not limited to the above-described embodiments, and various changes and modifications in form and detail may be made to the present disclosure by those skilled in the art without departing from the spirit and scope of the present disclosure, which are considered to fall within the protection scope of the present disclosure.

## Claims

1. A method for processing raw data from gene sequencing, comprising:
performing preprocessing and rectangle rotation on raw data obtained from gene sequencing to convert the raw data into a continuous single nanopore data structure with a specifiable length; and
performing compression on the data after the preprocessing and the rectangle rotation to eliminate invalid data.

2. The method according to claim 1, wherein the preprocessing and the rectangle rotation further comprise:
performing bit deletion on the data to satisfy byte alignment;
performing ordering and alignment on the data according to nanopore channels, and selecting specific channels for performing marking, so as to facilitate subsequent data checking; and
rectangle rotating the data ordered according to the nanopore channels into the continuous single nanopore data structure with the specifiable length.

3. The method according to claim 2, wherein the performing bit deletion on the data to satisfy byte alignment further comprises: performing bit reduction on the data in a manner of converting the data from absolute values to relative values.

4. The method according to claim 2, wherein the continuous single nanopore data structure with the specifiable length presents contextual information of the data to facilitate subsequent data compression.

5. The method according to claim 2, wherein the specifiable length is adjusted according to a size of hardware-integrated DDR particles and a throughput amount of a sequencing chip.

6. The method according to claim 5, wherein randomized processing is performed on the read and write addresses of the data during the rectangle rotation according to different DDR capacities and DDR bank sizes.

7. The method according to any one of claims 1 to 6, wherein the compression further comprises: adopting a compression encoding algorithm to eliminate the invalid data.

8. The method according to any one of claims 1 to 7, wherein the invalid data comprises invalid data without sequencing blocking and invalid data with sequencing blocking.

9. The method according to claim 8, wherein the compression further comprises: eliminating invalid continuous current value data when sequencing blocking exists and sequencing blocking does not exist.

10. The method according to any one of claims 1 to 9, wherein the compression further comprises: compressing the data after the preprocessing and the rectangle rotation to a state that the data can directly correspond to fragment sequence data of a base type.

11. The method according to any one of claims 1 to 10, wherein after performing compression on the data, caching is performed on the compressed data for back end resolving.

12. A nucleic acid sequencing method, comprising:
obtaining data of a current signal passing through a nanopore;
performing processing on the obtained data of the current signal according to the method for processing raw data from gene sequencing in any one of claims 1 to 11;
performing resolving according to the compressed data, and determining types of bases passing through the nanopore chronologically; and
determining a nucleic acid sequence according to the determined types of the bases passing through the nanopore chronologically.

13. An apparatus for processing raw data from gene sequencing, comprising:
a data preprocessing unit configured to perform preprocessing and rectangle rotation on raw data obtained from gene sequencing to convert the raw data into a continuous single nanopore data structure with a specifiable length; and
a data compressing unit configured to perform compression on the data after the preprocessing and the rectangle rotation to eliminate invalid data.

14. A nucleic acid sequencing system, comprising:
a current acquiring apparatus configured to obtain data of a current signal passing through a nanopore;
the apparatus for processing raw data from gene sequencing according to claim 13, configured to perform processing on the obtained data of the current signal;
a base type determining apparatus configured to perform resolving according to the compressed data after processing by the apparatus for processing raw data from gene sequencing, and to determine types of bases passing through the nanopore chronologically; and
a nucleic acid sequence determining apparatus configured to determine a nucleic acid sequence according to the types of the bases chronologically passing through the nanopore determined by the base type determining apparatus.

15. A non-transitory computer-readable storage medium for storing a computer program, the computer program comprising instructions that, when executed by a processor of an electronic device, cause the electronic device to perform the method for processing raw data from gene sequencing according to any one of claims 1 to 11 or the nucleic acid sequencing method according to claim 12.
